# EUROPÄISCHE PATENTSCHRIFT

(11) **EP 2 566 947 B1**
(45) Veröffentlichungstag und Bekanntmachung des Hinweises auf die Patenterteilung: **24.06.2015**
(21) Anmeldenummer: 12723087.8
(22) Anmeldetag: 05.04.2012
(51) Int. Cl.: C12M 1/107, C12M 1/113

(54) **VERFAHREN UND VORRICHTUNG ZUR HERSTELLUNG VON BIOGAS AUS GÄRSUBSTRAT**
PROCESS AND APPARATUS FOR PRODUCTION OF BIOGAS FROM FERMENTATION SUBSTRATE
PROCÉDÉ ET DISPOSITIF DE FABRICATION DE BIOGAZ À PARTIR D'UN SUBSTRAT DE FERMENTATION

(30) Priorität: 09.04.2011 DE 102011016604
(43) Veröffentlichungstag der Anmeldung: 13.03.2013
(73) Patentinhaber: DGE Dr.-Ing. Günther Engineering GmbH, 06886 Wittenberg (DE)
(72) Erfinder: GÜNTHER, Lothar, 82538 Geretsried (DE)
(74) Vertreter: Tragsdorf, Bodo
(86) Internationale Anmeldenummer: PCT/DE2012/100092
(87) Internationale Veröffentlichungsnummer: WO 2012/139563

(56) Entgegenhaltungen:
- EP-A1- 0 007 168
- EP-A1- 1 777 290
- WO-A1-2008/049613
- DE-A1- 4 341 713
- DE-A1- 4 423 099
- DE-A1-102006 030 773
- DE-A1-102007 007 510
- DE-A1-102007 037 202
- DE-B3-102007 048 565
- US-A1- 2010 206 791
- US-A1- 2010 311 111

## Beschreibung

Die Erfindung bezieht sich auf ein Verfahren zur Herstellung von Biogas aus Gärsubstrat, in mindestens einem Fermenter. Ferner ist eine zur Durchführung des Verfahrens geeignete Vorrichtung Gegenstand der Erfindung.

Die Herstellung von Biogas erfolgt in an sich bekannter Weise in einem oder mehreren Reaktoren bzw. Fermentern, die mesophil (Temperaturen unterhalb von 45 °C) oder thermophil (Temperaturen 45 bis 80 °C) betrieben werden können.

Unter Gärsubstrat ist Biomasse als organische Substanz zu verstehen, die durch Mensch, Tier und Pflanze entsteht, wie beispielsweise als Wirtschaftsdünger (Gülle, Mist), nach-wachsende Rohstoffe und biologische Abfallmaterialien.

Unter den Begriff Biomasse fällt auch Klärschlamm, wenn dieser aus organisch belasteten Ab- oder Prozesswässern besteht und für eine anaerobe Umsetzung geeignet ist. Ein derartiger Klärschlamm muss einen CSB-Anteil von über 5.000 mg/l haben. "CSB" ist die Abkürzung für "Chemischen Sauerstoffbedarf", wobei im Rahmen einer CSB-Messung ermittelt wird, wie viel Sauerstoff die chemischen Faulungs-/Reinigungsprozesse im Abwasser verbrauchen.

Zur Herstellung bzw. Erzeugung von Biogas finden während der Umsetzung bzw. Fermentation unterschiedliche biologische Abbauprozesse statt, als Hydrolyse, Versäuerung, Essigsäurebildung und Methanbildung. Die durch Bakterien verursachten Abbauprozesse können unter aeroben oder anaeroben Bedingungen stattfinden. Das am häufigsten angewendete Verfahren ist die Nassfermentation (Trockensubstanzgehalt TS < 15 % und Wassergehalt > 85 %).

Die Erzeugung von Biogas kann nach unterschiedlichen Verfahrensweisen erfolgen. Wünschenswert ist, ein Biogas mit einem hohen Methangehalt zu erhalten, da mit steigendem Anteil an Methan im hergestellten Biogas sich die Kosten für die nachträgliche Reinigung bzw. Aufarbeitung zu Methangas verringern.

Außer Methan enthält Biogas noch bis zu 60 Vol.-% CO₂, bis zu 5 Vol.-% Stickstoff, bis zu 2 % Sauerstoff, bis zu 2 % Wasserstoff, bis zu 2 Vol.-% H₂S, bis zu 8 Vol.-% H₂O und bis zu 1 Vol.-% Ammoniak bzw. Ammoniumverbindungen sowie ggf. noch andere organische Substanzen im ppm-Bereich. Die pH-Werte bei den Fermentationsprozessen liegen im Bereich von bei 7,7 bis 8,4, dies vor allem deshalb, weil die Methanbakterien im alkalischen Bereich bessere Ergebnisse erzielen. Der Gärprozess im Fermenter erfolgt üblicherweise bei Substrattemperaturen von ca. 40 °C.

Biomasse enthält Stickstoff (N), sodass während der Vergärung Ammonium (NH₄⁺) als Kation freigesetzt wird. Ammonium bildet bekanntlich ein Dissoziationsgleichgewicht mit Ammoniak, das vom pH-Wert und der Temperatur abhängig ist. Der Anteil an Ammoniak erhöht sich mit zunehmendem pH und steigender Temperatur. Mit steigendem Ammoniakgehalt verringert sich die Methanbildung während der Fermentation.

Ein genereller Nachteil der bekannten Verfahren sind zu geringe Ausbeuten an Methan bei der Umsetzung von Biomasse zu Biogas, insbesondere die zu geringen Methankonzentrationen bei der biologischen Umsetzung, sowie die relativ hohen Anteile an Schwefelwasserstoff und Ammoniak im erzeugten Biogas.

Bekannt ist, Gärsubstrat nach Beendigung des Gärprozesses in eine feste und flüssige Phase zu trennen und zumindest eine Teilmenge der flüssigen Phase einem neuen Ansatz an Bioabfall zuzusetzen, um die anaerobe Gärung wieder anzustoßen (z.B. DE 44 23 099 A1).

Der Fermentations- bzw. Gärprozess ist äußerst komplexer Natur. Das anfallende Gärsubstrat, das ein Gemisch aus Gärflüssigkeit und Feststoffen ist, enthält u.a. gelöste organische Verbindungen, die über den CSB-Gehalt mit 2.000 bis 20.000 mg/l oder höher liegen und physikalisch gebundenes CO₂ sowie Ammoniumverbindungen (NH₄⁺) und NH₃, das auch als Ammoniumcarbonat, Ammoniumcarbamat, Ammonium usw. chemisch gebunden vorliegt. Der Anteil von Ammoniumverbindungen im Gärsubstrat ist abhängig von der Art der Biomasse. Gülle und Jauche enthalten beispielsweise einen relativ hohen Anteil an Ammoniak und chemisch gebundenem Stickstoff (3 bis 8 g/l).

Bei Biomasse auf Basis pflanzlicher Rohstoffe entsteht Ammoniak bei der Vergärung N-haltiger Anteile. Gülle und Jauche enthalten zudem einen relativ hohen Anteil an Harnstoff (Urin), wodurch während des Gärprozesses zusätzlich noch Ammoniak freigesetzt wird. Da in der Praxis häufig Biomassegemische eingesetzt werden, die pflanzliche und tierische Rohstoffe in unterschiedlichen Kombinationen und Zusammensetzungen enthalten, ist die während der Vergärung tatsächlich auftretende Ammoniakfreisetzung so gut wie nicht vorherbestimmbar.

Aus der DE 43 41 713 A1 ist ein Verfahren zur Entsorgung von Biomasse bekannt, wobei während der Verweildauer der Biomasse im Vergärungstank ein Teilstrom an Biomasse entnommen und in einem geschlossenen Kreislauf durch einen ersten Wärmetauscher und einen zweiten Wärmetauscher gepumpt und erwärmt wird. Der erwärmte Teilstrom gelangt in einen Ammoniakabscheider, in dem unter Vakuum aus dem Teilstrom an Biomasse ein Ammoniak-Wasser-Dampf-Gemisch ausgetrieben wird. Die ammoniakreduzierte Biomasse wird wieder in den Fermenter zurückgeführt.

Der Erfindung liegt die Aufgabe zugrunde, ein Verfahren zur Herstellung von Biogas aus Gärsubstrat zu schaffen, bei dem während der Vergärung eine erhöhte Bildung an Methan ermöglicht wird. Ferner soll eine zur Durchführung des Verfahrens geeignete Vorrichtung bereitgestellt werden. Erfindungsgemäß wird die Aufgabe durch die im Anspruch 1 angegebenen Verfahrensmerkmale gelöst. Vorteilhafte Weiterbildungen der Verfahrensweise sind Gegenstand der Ansprüche 2 bis 12.

Während der Verweildauer des Gärsubstrates im Fermenter wird die Ammoniakkonzentration im Gärsubstrat überwacht. Der Ammoniakgehalt im Gärsubstrat liegt während der Fermentation zwischen 0,6 und 0,9 g/l. Bei Erreichen eines Grenzwertes von 1 g/l wird Gärsubstrat ausgekreist und in einem geschlossenen Kreislauf durch einen ersten Wärmetauscher gepumpt. In diesem wird das Gärsubstrat von seiner Ausgangstemperatur von ca. 40 °C auf Temperaturen von bis zu 110 °C erwärmt und anschließend einem weiteren, zweiten Wärmetauscher zugeführt und in diesem weiter bis auf maximal 180 °C erwärmt. Durch den Pumpvorgang wird das ausgekreiste Gärsubstrat auf einen Druck von 1,5 bis 10 bar, vorzugsweise 2 bis 6 bar, verdichtet. Nach dem zweiten Wärmetauscher gelangt das komprimierte heiße Gärsubstrat in einen geschlossenen Behälter oder Abscheider. Im Behälter oder Abscheider werden durch eine Entspannung oder bei Aufrechterhaltung des Behälterinnendruckes aus dem Gärsubstrat CO₂, Ammoniak, Spuren an H₂S und Wasserdampf freigesetzt. Diese werden als Gasgemisch über eine in den Behälter eingebundene, druckgeregelte Abgasleitung abgeführt und gereinigt, wobei in einem Wäscher Ammoniak und H₂S als Dünger chemisch gebunden werden;

Das ammoniakreduzierte Gärsubstrat wird über eine Leitung aus dem Behälter abgeführt und nach erfolgte Abkühlung auf Fermentertemperatur wieder dem im Fermenter befindlichen Gärsubstrat zur weiteren Vergärung zugeführt.

Die Menge an zugeführtem Gärsubstrat und abgeführten ammoniakreduziertem Gärsübstrat sollten so aufeinander abgestimmt werden, dass im Behälter ständig ein Füllvolumen an Gärsubstrat von mindestens 20 % vorliegt.

Der Begriff Fermenter im Sinne der Erfindung umfasst auch Nachgärer und Gärrestlager, Einzeln oder als Komponenten einer Biogasanlage, da in diesen ebenfalls ein Gärprozess unter Bildung von Biogas stattfindet.

Die Teilmenge an Gärsubstrat, die stündlich ausgekreist wird, sollte ca. 0,1 bis 1 % des Fermentervolumens betragen.

Die Auskreisung einer Teilmenge an Gärsubstrat erfolgt erst nach Erreichen eines Grenzwertes für die Freisetzung einer die Methanbildung hemmenden Menge an Ammoniak.

Als Gärsubstrat können alle unter den Begriff Biomasse fallenden Stoffe bzw. Materialien eingesetzt werden, in analoger Weise wie bei den bisher bekannten Verfahren zur Erzeugung von Bio- oder Klärgas. Diese können in unterschiedlicher Konsistenz vorliegen und werden erforderlichenfalls noch auf den gewünschten TS-Gehalt eingestellt. Beispielsweise liegt der TS-Gehalt von flüssigem Gärsubstrat, wie z.B. Gülle, unter 3 %.

Beim Einsatz von Biomassen auf Basis pflanzlicher Rohstoffe oder Gemischen aus diesen liegt der TS-Gehalt des Substrates bzw. -gemisches oberhalb von 3.

Mit der vorgeschlagenen Verfahrensweise kann die Grundlast an Ammonium gegenüber bekannten Biogasanlagen von ca. 2 bis 3 g/l auf 1 bis 1,5 g/l oder auch darunter abgesenkt werden.

Durch die Auskreisung von bereits im Gärstadium vorliegendem Gärsubstrat, bei dem die übliche Freisetzung von Ammonium noch nicht erfolgt ist und diese erst gezielt, außerhalb des Fermenters, im Behälter der Kreislaufschleife vorgenommen wird, wird in den Fermenter ammoniakreduziertes Gärsubstrat zurückgeführt und dadurch die Gefahr, dass im Fermenter eine die Methanbildung hemmende Ammoniumkonzentration entsteht, beseitigt. Dadurch erhöhen sich die Ausbeute an Biogas und die Methanbildungsgeschwindigkeit. Eine erhöhte Methanbildungsgeschwindigkeit kann auch höhere Konzentrationen an Methan im Biogas ergeben.

In ersten Versuchen konnten um ca. 8 bis 15 % höhere Ausbeuten an Biogas erzielt werden.

Aus dem Fermenter wird verfahrensabhängig entweder kontinuierlich oder nur absatzweise eine bestimmte Teilmenge an Gärsubstrat ausgekreist und als ammoniakreduziertes Gärsubstrat wieder zurückgeführt.

Da während der Fermentation die Methanbildung nicht mehr gehemmt wird, verkürzt sich die Fermentationsdauer um ca. 30 % bis 45 %. Ein weiterer Vorteil ist darin zu sehen, dass im Gärsubstrat enthaltener Schwefelwasserstoff zu über 80 % mit dem Ammoniak entweicht. Demzufolge weist unter den erfindungsgemäßen Maßnahmen im Fermenter erzeugtes Biogas einen geringeren Anteil an Schwefelwasserstoff auf.

Ein zusätzlicher, vorteilhafter Nebeneffekt besteht noch darin, dass nach Beendigung der Fermentation anfallender Gärrest im Vergleich zu ansonsten anfallendem Gärrest eine geringere Ammonium/Ammoniakbelastung aufweist und somit eine deutlich verminderte Geruchsbelästigung. Diese spielt vor allem beim Ausbringen von Gärrest auf landwirtschaftliche Nutzflächen eine nicht unbedeutende Rolle.

Aus Untersuchungen Dritter ist bekannt, dass unter folgenden Bedingungen mit einer Hemmung der Methanbildung zu rechnen ist.

| Konzentration an NH₄⁺ | pH-Wert |
|---|---|
| 6 g/l | 6,7 |
| 3 g/l | 7,0 |
| 1 g/l | 7,5 |

Als Grenzwert für diese Bedingungen wurde ein sich einstellender Anteil von freiem Ammoniak im Gärsubstrat von 100 mg/l angegeben. Eigene Untersuchungen und Berechnungen haben ergeben, dass bereits ab einem Wert von etwa 15 mg/l für freies Ammoniak im Gärsubstrat bereits zu einer deutlichen Hemmung der Methanbildung führen. Demzufolge muss damit gerechnet werden, dass die Hemmung der Methanbiologie bereits deutlich früher eintritt. Bei einer mesophilen Betriebsweise ergibt sich unter diesen Bedingungen ein Ammoniakgehalt im Biogas von 30 ppm. Das entspricht in etwa 15 mg/l freiem Ammoniak im Gärsubstrat bei einem pH-Wert von 7,2.

Der NH₄⁺-Gehalt kann während des Gärprozesses erheblich schwanken. Über die völlig unterschiedlichen und überlagerten Bedingungen des Fermentationsprozesses ist es praktisch unmöglich die Dissoziationsgleichgewichte zuverlässig vorausberechnen, da neben den ständig wechselnden Zusammensetzungen der organischen Säure noch die gelösten Nährstoffionen einen Einfluss ausüben. Weiter wird dies dadurch erschwert, da das Biogas vor der Messung konditioniert, d.h. gekühlt wird und dabei Wasser kondensiert. Das kondensierte Wasser bindet Ammoniak. Der im kondensierten Wasser gelöste Anteil an Ammoniak muss hier mit berücksichtigt werden.

Im Rahmen von Versuchen wurde gefunden, dass allein über die Messung der Ammoniakkonzentration im Biogas ein zuverlässiger Rückschluss auf die im Gärsubstrat freie gelöste Menge an Ammoniak über das Dampf-Flüssigkeitsgleichgewicht in Vebindung mit dem Henry-Koeffizienten besteht.

Als vorgegebener Grenzwert für die die Methanbildung hemmende Menge an Ammoniak kann von einer im Biogas gemessenen Konzentration an Ammoniak von 30 ppm ausgegangen werden. Dieser Grenzwert stellt einen allgemeinen Richtwert dar, von dem unter bestimmten Bedingungen auch abgewichen werden kann.

Durch dosierten Zusatz von ammoniakreduziertem Gärsubstrat in den oder die Fermenter lässt sich der Ammoniakgehalt während der Fermentation so einstellen, dass günstige Bedingungen für die Methanbildung und deren Geschwindigkeit herrschen. Der Ammoniakgehalt im Gärsubstrat während der Fermentation soll zwischen 0,6 bis 0,9 g/l liegen. Eine vollständige Entfernung von Ammonium ist nicht gewünscht, da die zur Vergärung wirksamen Mikroorganismen diesen als Nahrung benötigen.

Während des Gärprozesses wird daher die Ammoniakkonzentration überwacht, vorzugsweise über eine Ammoniakmessung im Biogas.

Die Messung bzw. Überwachung der Ammoniakkonzentration erfolgt mittels handelsüblicher Gasmessgeräte.

Der erforderliche Systemdruck innerhalb der Kreislaufschleife wird mittels einer Pumpe erzeugt. Zur Erwärmung/Erhitzung wird das Gärsubstrat durch mehrere Wärmetauscher gepumpt, die als Rohrbündelwärmetauscher ausgeführt sind, wobei als Wärmeträger Thermalöl und/oder rückgeführtes ammoniakreduziertes Gärsubstrat verwendet werden. Dadurch wird eine energetisch vorteilhafte Verfahrensweise erreicht.

Die vorgeschlagene Verfahrensweise kann auch zur Herstellung von Biogas mittels mehrerer Fermenter genutzt werden, die parallel oder in Reihe geschaltet sein können. Im Gärzustand befindliches Gärsubstrat kann wahlweise aus dem ersten Fermenter und/oder zu Beginn und/oder während der Vergärung in einem nachgeschalteten Fermenter ausgekreist werden. Ammoniakreduziertes Gärsubstrat kann zur Weiterführung der anaeroben Vergärung in einen der Fermenter, oder auch aufgeteilt auf mehrere, zurückgeführt werden.

Bei Einsatz mehrerer Fermenter wird der Ort der Auskreisung von Gärsubstrat, erster oder nachfolgender Fermenter, in Abhängigkeit von der Geschwindigkeit der Ammoniakbildung im Gärsubstrat bestimmt.

Über die abgeführte Kreislaufmenge an Gärsubstrat und die wieder zurückgeführte Menge an ammoniakreduziertem Gärsubstrat lässt sich die Ammoniumkonzentration im Fermenter bzw. in den Fermentern beeinflussen.

Aus dem Behälter bzw. Abscheider abgeführtes ammoniakreduziertes Gärsubstrat wird zur Abkühlung auf Fermentertemperatur durch einen dritten Wärmetauscher geleitet. Vorzugsweise wird im dritten Wärmetauscher erwärmtes Wärmeträgermedium über eine Kreislaufleitung dem ersten Wärmetauscher zugeführt und gelangt wieder zurück in den dritten Wärmetauscher. Für die Wärmeübertragung wird nur ein Wärmeträgermedium eingesetzt.

Der zweite Wärmetauscher wird separat beheizt, beispielsweise mit Thermalöl als Wärmeträger.

Die Verweildauer des Gärsubstrates im ersten Fermenter ist u.a. von der Zusammensetzung des Gärsubstrates, insbesondere vom TS-Gehalt, abhängig.

Der TS-Gehalt des Gärsubstrates kann durch Zugabe von Gärflüssigkeit und/oder Wasser bis auf einen Wert von unter 10% eingestellt werden.

Gemäß einer alternativen Ausführungsvariante kann das ausgekreiste Gärsubstrat vor der Einleitung in den ersten Wärmetauscher in eine flüssige und höher konzentrierte Phase getrennt werden. Nachfolgend wird nur die Ammonium/Ammoniakkonzentration der flüssigen Phase reduziert. Hierbei besteht kein wesentlicher Unterschied im Vergleich zur vorstehend beschriebenen-Behandlung von Gärsubstrat. Abschließend werden die flüssige Phase und die höher konzentrierte Phase wieder zusammengeführt und ggf. noch vor der Rückführung in den Fermenter vermischt.

Die bei der Abscheidung von Ammoniak gebildeten Brüden werden über eine Leitung druckgeregelt aus dem Behälter bzw. Abscheider abgeführt. Die Brüden werden kondenkondensiert, wobei Ammoniakwasser und Ammoniumcarbonat als wässrige Lösung entstehen. Da nur ca. 70 bis 90 % des Ammoniaks zu Ammoniakwasser und (2 NH₃ + CO₂) zu Ammoniumcarbonat oder deren Verbindungen reagieren, wird der Rest an Ammoniak mit Säure, z.B. Schwefelsäure, Phosphorsäure oder Salpetersäure, chemisch gebunden. Ammoniumcarbonat kann als wässrige Lösung zur Bodendüngung in der Landwirtschaft eingesetzt werden.

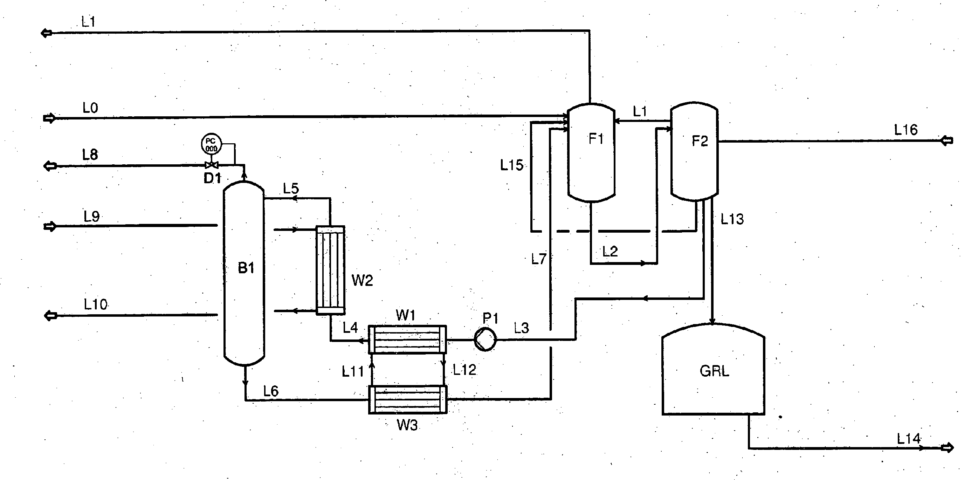

Die Erfindung wird nachstehend anhand der Zeichnung und einem Beispiel erläutert. Die zugehörige Zeichnung zeigt eine Anlage zur Durchführung des Verfahrens in vereinfachter schematischer Darstellung.

In einer Biogasanlage werden pro Jahr 10.000 m³ Rindergülle mit einer Zusammensetzung von

| | |
|---|---|
| TS-Gehalt .. | 10,5 % |
| Organischer Anteil | 76,5% |
| NH₄⁺ ges. | 4,5 g/l, davon gelöst 1,5 g/l |

als Gärsubstrat eingesetzt.

Die Vergärung erfolgt in zwei in Reihe geschalteten Fermentern F1 und F2, thermophil bei einer Temperatur von 55 °C und pH-Werten von 7,2 bis 7,6.

Die beiden Fermenter F1 und F2 besitzen ein Fermentervolumen von 3.000 m³ und sind über eine Transportleitung L2 miteinander verbunden, über die Gärsubstrat nach einer bestimmten Gärzeit vom ersten in den zweiten Fermenter gepumpt wird. In beiden Fermentern ist eine biologische Entschwefelung, die mit Luft oder Sauerstoff arbeitet, integriert. Die Zuführung von Luft oder Sauerstoff erfolgt über die Leitung L16. Durch die Entschwefelung wird der Gehalt an Schwefelwasserstoff auf unter 100 ppm im Biogas der beiden Fermenter reduziert.

Der erste Fermenter F1 wird mit stündlich ca. 1,16 m³ frischer Rindergülle über die Leitung L0 beschickt. Über die Leitung L15 werden kontinuierlich aus dem zweiten Fermenter F2 1 m³/h an vergorener Gülle in den ersten Fermenter F1 zurückgeführt und über die Leitung L7 4 m³/h an ammoniakreduzierter Gülle, wie nachfolgen noch erläutert.

Im ersten Fermenter F1 stellt sich somit ein TS-Gehalt von etwa 3 % ein.

Nach einer mittleren Fermentationsdauer von 12 Tagen sind ca. 70 % des organischen Anteiles des zugeführten Gärsubstrates biologisch abgebaut. Diese gelangen über die Leitung L2 in den zweiten Fermenter F2. In den ersten Fermenter F1 wird dann die erforderliche Menge an Gülle nachdosiert.

Das im ersten Fermenter F1 nach 12 Tagen anfallende Gärsubstrat (Temperatur von 55 °C) hat folgende Zusammensetzung:

| | |
|---|---|
| NH₄⁺ | 4,4 g/l, davon gelöster Anteil 2,6 |
| CO₂ | 7,2 g/l |
| H₂S | 65 mg/l |
| TS-Gehalt | 2,0%. |

Im über die Leitung L 1 aus dem Fermenter F1 abgezogenen Biogas wird die Ammoniakkonzentration im gekühlten Biogas und im Kondensat gemessen.

Das erzeugte Biogas hat folgende trockene Zusammensetzung:

| | |
|---|---|
| CH₄ | 58 Vol.-% |
| CO₂ | 40,8 Vol.% |
| O₂ | 0,2 Vol.-% |
| N₂ | 0,9 Vol.-% |
| H₂ | 850 ppm |
| H₂S | 75 ppm |
| NH₃ | 25 ppm. |

Bei Erreichen eines Grenzwertes von 30 ppm an Ammoniak im Biogas wird über die Leitung L3 vergorenes Gärsubstrat in einer Menge von 4 m³/h aus dem zweiten Fermenter F2 ausgekreist.

Die Auskreisung von Gärsubstrat aus dem zweiten Fermenter F2 erfolgt über eine mit dem Fermenter F1 verbundene Kreislaufschleife, bestehend aus der Leitung L3 zur Substratzuführung, der Kreislaufpumpe P1, dem ersten Wärmetauscher W1, der Verbindungsleitung L4 vom ersten W1 zum zweiten Wärmetauscher W2, der Leitung L5, dem Behälter bzw. Abscheider B1, der Leitung L6, die vom Behälter B1 zum dritten Wärmetauscher W3 führt und der vom Wärmetauscher W3 abgehenden Leitung L7, über die ammoniakreduziertes Gärsubstrat in den Fermenter F1 zurückgeführt wird.

Über die am Kopf des Behälters B1 angeordnete Leitung L8 werden die im Behälter B1 entstehenden Brüden, Gemisch aus NH₃, CO₂, H₂S und Wasserdampf, zur weiteren Behandlung abgeführt, wobei in diese Leitung ein Druckbegrenzungsventil D1 eingebunden ist.

Die beiden Wärmetauscher W1 und W3 sind mit Leitungen L11 und L12 verbunden, in denen das Wärmeträgermedium, z. B. Wasser, zirkuliert. Der zweite Wärmetauscher W2 wird separat mittels Thermalöl als Wärmeträger beheizt, das über die Leitung L9 zu- und über die Leitung L10 abgeführt wird.

Anlagentechnisch ist die Kreislaufschleife als eigenständige Baugruppe ausgeführt, die auch an Fermenter bereits bestehender Biogasanlagen angeschlossen werden kann. Über die Fahrweise mittels Kreislaufpumpe P1 mit einer Frequenzregelung kann jede beliebige Kreislaufmenge von 1- bis 6 m³/h eingestellt werden. Damit kann die Kreislaufschleife bzw. -anlage auch ständig mit geringer Leistung in Betrieb sein.

Im Betriebszustand wird über die Pumpe P1 Gärsubstrat in einer Menge von 4 m³/h über die Leitung L3 ausgekreist und durchströmt den Wärmetauscher W1. Dabei wird das Gärsubstrat von 55 °C bis auf 110°C erwärmt, mittels Wasser oder Thermalöl als Wärmeträger, das über die Leitungen L11 und L12 unter Druck zwischen den beiden Wärmetauschern W1 und W3 im Kreislauf gefahren wird. Die Aufheizung des Wärmeträgers erfolgt im dritten Wärmetauscher W3 über das aus dem Behälter B1 über die Leitung L6 abgeführte heiße ammoniakreduzierte Gärsubstrat.

Das ausgekreiste Gärsubstrat wird über die Leitung L4 zum zweiten Wärmetauscher W2 gefördert und in diesem bis auf 125 °C erwärmt. Der erforderliche Wärmeeintrag von ca. 200 kW erfolgt mittels aufgeheiztem Thermalöl, das über die Leitung L9 zu- und über die Leitung L10 abgeführt wird.

Das heiße Gärsubstrat wird unter Druck über die Leitung L5 in den Abscheidebehälter B1 geleitet. Der Systemdruck wird auf 2,6 bar eingestellt, wobei der Druck über das in die Leitung L8 eingebundene Druckregelventil D1 geregelt wird. Die zugeführte und abgeführte Kreislaufmenge an Gärsubstrat wird so geregelt, dass im Behälter B1 ständig mindestens 40 % des Behältervolumens mit heißem Gärsubstrat gefüllt sind.

Aus dem Gärsubstrat werden im Behälter B1 CO₂, Ammoniak sowie Spuren an H₂S und Wasserdampf freigesetzt, die als Brüden über die Leitung L8 entweichen, wobei stündlich 2 kg/h Ammoniak, 7,4 kg/h CO₂, 0,054 kg/h H₂S und 66,6 kg/h Wasserdampf ausgetragen werden.

Am Boden des Behälters B1 werden über die Leitung L6 kontinuierlich 4 m³/h ammoniakreduziertes Gärsubstrat (TS-Gehalt 2 %) mit folgender Zusammensetzung ausgetragen:

| | |
|---|---|
| NH₄⁺ | 1,9 g/l, davon 1,0 g/l gelöst |
| CO₂ | 2,7 g/l |
| H₂S | 5 mg/l. |

Das heiße ammoniakreduzierte Gärsubstrat wird zur Abkühlung auf Fermentertemperatur durch den Wärmetauscher W3 geleitet und abschließend über die Leitung L7 dem ersten Fermenter F1 zugeführt.

Die über die Leitung L8 abgeschiedenen Brüden werden gereinigt, wobei in einem Wäscher Ammoniak und H₂S als Dünger chemisch gebunden werden.

Nach Beendigung der Fermentation im zweiten Fermenter F2 wird angefallener Gärrest über die Leitung L13 dem Gärrestlager GRL zugeführt, das in bestimmten Abständen über die Leitung L14 entleert wird. Der abgeführte Gärrest besitzt im Vergleich zu aus bisher bekannten Biogasanlagen stammendem Gärrest eine deutlich geringere Ammoniakkonzentration.

Über die ausgekreiste und wieder zurückgeführte Kreislaufmenge an Gärsübstrat lässt sich der Ammoniumgehalt im Fermenter einstellen.

Das erzeugte Biogas weist einen deutlich geringeren Anteil an H₂S und NH₃ auf. Der Anteil an H₂S liegt jetzt unter 75 ppm und beim NH₃ unter 25 ppm.

Gemäß diesem Beispiel können in den beiden Fermentern 310.000 m³ Biogas oder kontinuierlich 36 m³/h trockenes Biogas mit 58 Vol.% CH₄, 40,8 Vol.-% CO₂, 0,085 Vol.-% H₂, 75 ppm H₂S und 25 ppm NH₃ erzeugt werden.

Aus den eingesetzten 10.000 m³ Rindergülle pro Jahr fallen als Nebenprodukt 18 t Ammonium oder 17 t Ammoniak an.

### Beispiel 2

In einer Biogasanlage werden zur Erzeugung von Biogas (1.000 m³/h) 4.950 kg Biomasse (Maissilage, TS-Gehalt 32 %) eingesetzt.

Die Fermentation erfolgt in vier in Reihe geschalteten Fermentern, wobei die beiden letzten Fermenter auch parallel betrieben werden können.

Der erste Fermenter hat ein Volumen von 1.500 m³ und die drei nachgeschalteten Fermenter haben jeweils ein Volumen von 3.000 m³.

Der erste Fermenter wird mit einer Menge von 4.950 kg/h an Maissilage mit einem TS-Gehalt von 5 % beschickt. Die Fermentation erfolgt mesophil bei Temperaturen von ca. 38 °C und pH-Werten von 7,1 bis 5,4 abfallend.

Nach einer mittleren Fermentationsdauer von 2 Tagen sind im ersten Fermenter ca. 30% an organischer Substanz abgebaut. Das so vergorene Gärsubstrat (Temperatur 40 °C) mit einem TS-Gehalt von 3,5 % gelangt in den nachgeschalteten zweiten Fermenter und hat folgende Zusammensetzung:

| | |
|---|---|
| NH₄⁺ | <1,5 g/l gelöster Anteil |
| CO₂ | 5,8 g/l |
| H₂S | 50 mg/l. |

Der Ammoniakgehalt im aus dem ersten Fermenter abströmenden Biogas wird nicht gesondert überwacht. Nach einer mittleren Verweilzeit von 4 Tagen im zweiten Fermenter wird das Gärsubstrat vom zweiten Fermenter in den nachfolgenden dritten Fermenter geleitet. Während dieser Verweilzeit hat sich die organische Substanz um weitere 50 % abgebaut, durch Bildung von Biogas. Im Gärsubstrat reduziert sich der TS-Gehalt auf 1,8 %. Danach wird das Gärsubstrat zum weiteren biologischen Abbau in den vierten Fermenter geleitet.

An den zweiten oder dritten Fermenter der Biogasanlage ist eine Kreislaufschleife analog der in der Zeichnung gezeigten Ausführung angeschlossen.

Hier werden kontinuierlich über die Leitung L3 12 m³/h Gärsubstrat (TS-Gehalt 3,5 %) abgepumpt, im Wärmetauscher W1 von 40 auf 110°C und im zweiten Wärmetauscher W2 weiter bis auf 125°C erwärmt. Danach gelangt das heiße Gärsubstrat über die Leitung L5 in den Abscheidebehälter B1. Unter Einhaltung eines Systemdruckes von 2,6 bar entweichen über die Leitung L8 aus dem Gärsubstrat frei gesetztes CO₂ und Ammoniak, sowie Spuren an H₂S und Wasserdampf.

Damit werden über die Leitung L8 kontinuierlich 5,64 kg/h Ammoniak, 62,4 kg/h CO₂, 0,54 kg/h H₂S und 66,6 kg/h Wasserdampf ausgetragen. Pro m³ Kreislaufmenge werden etwa 0,5 kg Ammonium aus dem Gärsubstrat abgeschieden.

Aus dem Abscheider B1 wird über die Leitung L6 ammoniakreduziertes Gärsubstrat (TS-Gehalt 4 %) folgender Zusammensetzung zum Wärmetauscher W3 geleitet:

| | |
|---|---|
| NH₄⁺ | <1,0 g/l |
| CO₂ | 0,6 g/l |
| H₂S | 5 mg/l. |

Über die Leitung L7 wird auf 40 °C abgekühltes ammoniakreduziertes Gärsubstrat in einer Menge von 12 m³/h wieder dem ersten Fermenter zugeführt.

Von den im Wärmetauscher W2 eingetragenen 240 kW an Wärme werden 180 kW mit dem warmen ammoniakreduziertem Gärsubstrat wieder in den ersten Fermenter zurückgeführt, wodurch die Fermenterheizung entlastet wird.

Die über die Leitung L8 abgeschiedenen Brüden werden gereinigt, wobei in einem Wäscher Ammoniak und H₂S als Dünger chemisch gebunden werden.

Über die mittels der Pumpe P1 einstellbare Kreislaufmenge an Gärsubstrat kann der Ammoniumgehalt im ersten Fermenter gezielt eingestellt werden.

Ohne Auskreisung einer Teilmenge an Gärsubstrat und Reduzierung des Ammoniakgehaltes würde im Gärsubstrat der Fermenter ein höherer Ammonium/Ammoniakanteil vorliegen, durch den die Biogasbildung gehemmt wird.

## Patentansprüche

1. Verfahren zur Herstellung von Biogas aus Gärsubstrat in mindestens einem Fermenter, wobei während der Verweildauer des Gärsubstrates im Fermenter (F1) Gärsubstrat ausgekreist und in einem geschlossenen Kreislauf durch einen ersten Wärmetauscher (W1) gepumpt und dabei bis zu 110 °C erwärmt und anschließend einem weiteren, zweiten Wärmetauscher (W2) zugeführt und weiter bis auf 180 °C erwärmt wird, und das erwärmte Gärsubstrat in einen geschlossenen Behälter oder Abscheider (B1) gepumpt wird, wobei in diesem Ammoniak ausgetrieben wird, und ammoniakreduziertes Gärsubstrat aus dem Behälter oder Abscheider (B1) abgeführt und wieder dem im Fermenter (F1) befindlichen Gärsubstrat zugeführt wird, **dadurch gekennzeichnet, dass** die Ammoniakkonzentration im Gärsubstrat im Dermenter (1) überwacht wird und der Ammoniakgehalt im Gärsubstrat während der Fermentation zwischen 0,6 und 0,9 g/l liegt und bei Erreichen eines Grenzwertes von 1 g/l Gärsubstrat ausgekreist wird, ausgekreistes Gärsubstrat vor der Einleitung in den geschlossenen Behälter oder Abscheider (B1) erwärmt und auf einen Druck von 1,5 bis 10 bar verdichtet wird und das heiße komprimierte Gärsubstrat in den Behälter oder Abscheider (B1) eingeleitet wird, wobei unter Aufrechterhaltung des Druckes oder Entspannung aus dem Gärsubstrat außer Ammoniak noch CO₂, Spuren an H₂S und Wasserdampf freigesetzt werden, die als Gasgemisch über eine in den Behälter oder Abscheider (B1) eingebundene Abgasleitung (L8) abgeführt und gereinigt werden, wobei in einem Wäscher Ammoniak und H₂S als Dünger chemisch gebunden werden.

2. Verfahren nach Anspruch 1, **dadurch gekennzeichnet, dass** die Menge an Gärsubstrat, die in den Behälter (B1) eingetragen wird und die Menge an ammoniakreduziertem Gärsubstrat, die aus dem Behälter (B1) abgeführt wird, so aufeinander abgestimmt werden, dass im Behälter (B1) ständig ein Füllvolumen an Gärsubstrat von mindestens 20 % vorliegt.

3. Verfahren nach einem der Ansprüche 1 oder 2, **dadurch gekennzeichnet, dass** die Vergärung in zwei oder mehreren parallel oder in Reihe geschalteten Fermentern erfolgt, wobei Gärsubstrat aus dem ersten und/oder zu Beginn und/oder während der Vergärung in einem nachgeschalteten Fermenter ausgekreist wird, und ammoniakreduziertes Gärsubstrat zur Fortsetzung der anaeroben Vergärung in mindestens einen der Fermenter zurückgeführt wird.

4. Verfahren nach einem der Ansprüche 1 bis 3, **dadurch gekennzeichnet, dass** die Überwachung der Ammoniakkonzentration im Fermenter durch eine Messung des Ammoniakgehaltes im aus dem Fermenter abgeführten Biogas vorgenommen wird.

5. Verfahren nach einem der Ansprüche 1 bis 4, **dadurch gekennzeichnet, dass** das ausgekreiste Gärsubstrat auf einen Druck von 2 bis 6 bar, verdichtet wird.

6. Verfahren nach einem der Ansprüche 1 bis 5, **dadurch gekennzeichnet, dass** über die abgeführte Kreislaufmenge an Gärsubstrat und die wieder zurückgeführte Menge an ammoniakreduziertem Gärsubstrat die Ammoniumkonzentration im Fermenter geregelt wird.

7. Verfahren nach einem der Ansprüche 1 bis 6, **dadurch gekennzeichnet, dass** aus dem Behälter (B1) abgeführtes ammoniakreduziertes Gärsubstrat zur Abkühlung auf Fermentertemperatur durch einen dritten Wärmetauscher (W3) geleitet wird.

8. Verfahren nach einem der Ansprüche 1 bis 7, **dadurch gekennzeichnet, dass** das im dritten Wärmetauscher (W3) erwärmte Wärmeträgermedium über eine Kreislaufleitung (L11, L12) dem ersten Wärmetauscher (W1) zugeführt und wieder zurück in den dritten Wärmetauscher (W3) gelangt.

9. Verfahren nach einem der Ansprüche 1 bis 8, **dadurch gekennzeichnet, dass** der zweite Wärmetauscher separat beheizt wird.

10. Verfahren nach einem der Ansprüche 1 bis 9, **dadurch gekennzeichnet, dass** die Verweildauer des Gärsubstrates im ersten Fermenter (F1) von der Zusammensetzung des Gärsubstrates, insbesondere vom TS-Gehalt, abhängig ist.

11. Verfahren nach einem der Ansprüche 1 bis 10, **dadurch gekennzeichnet, dass** der TS-Gehalt des Gärsubstrates durch Zugabe von Gärflüssigkeit und/oder Wasser bis auf einen Wert von unter 10 % eingestellt wird.

12. Verfahren nach einem der Ansprüche 1 bis 11, **dadurch gekennzeichnet, dass** das ausgekreiste Gärsubstrat vor der Einleitung in den ersten Wärmetauscher (W1) in eine flüssige und höher konzentrierte Phase getrennt wird, nachfolgend nur die Ammoniakkonzentration der flüssigen Phase reduziert wird und abschließend die flüssige Phase und die höher konzentrierte Phase wieder zusammengeführt werden.

## Claims

1. Process for the production of biogas from fermentation substrate in at least one fermenter, wherein fermentation substrate is separated out during the residence time of the fermentation substrate in the fermenter (F1) and pumped through a first heat exchanger (W1) in a closed circuit and heated up to 110°C in the process, and subsequently fed to an additional, second heat exchanger (W2) and thereafter heated up to 180°C, and the heated fermentation substrate is pumped into a closed vessel or separator (B1), wherein ammonia in said vessel or separator is expelled, and ammonia-reduced fermentation substrate is removed from the vessel or separator (B1) and returned to the fermentation substrate in the fermenter (F1), **characterized in that** the ammonia concentration in the fermentation substrate in the fermenter (1) is monitored and the ammonia content in the fermentation substrate during fermentation is between 0.6 and 0.9 g/l and is separated out when a limit value of 1 g/l fermentation substrate is reached, the fermentation substrate separated out is heated and compressed to a pressure of 1.5 to 10 bar before being conducted into the closed vessel or separator (B1) and the hot compressed fermentation substrate is conducted into the closed vessel or separator (B1), wherein, with the pressure maintained or released, CO₂, traces of H₂S and water vapor are released from the fermentation substrate in addition to ammonia and are removed as a gas mixture via an exhaust gas line (L8) integrated in the vessel or separator (B1) and purified, wherein ammonia and H₂S are chemically bound as a fertilizer in a scrubber.

2. Process according to claim 1, **characterized in that** the amount of fermentation substrate fed into the vessel (B1) and the amount of ammonia-reduced fermentation substrate removed from the vessel (B1), are coordinated in such a way that there is always a filling volume of fermentation substrate of at least 20% in the vessel (B1).

3. Process according to one of the claims 1 or 2, **characterized in that** fermentation takes place in two or several fermenters connected in parallel or series, wherein fermentation substrate from the first fermenter is separated out in a downstream fermenter and/or at the start of and/or during fermentation, and ammonia-reduced fermentation substrate is returned to at least one of the fermenters to continue the anaerobic fermentation.

4. Process according to one of the claims 1 to 3, **characterized in that** the ammonia concentration in the fermenter is monitored by measuring the ammonia content in the biogas removed from the fermenter.

5. Process according to one of the claims 1 to 4, **characterized in that** the fermentation substrate separated out is compressed to a pressure of 2 to 6 bar.

6. Process according to one of the claims 1 to 5, **characterized in that** the ammonia concentration in the fermenter is regulated via the amount of fermentation substrate in the circuit that has been removed and the amount of ammonia-reduced fermentation substrate that has been recirculated.

7. Process according to one of the claims 1 to 6, **characterized in that** the ammonia-reduced fermentation substrate removed from the vessel (B1) for cooling to fermenter temperature is conducted through a third heat exchanger (W3).

8. Process according to one of the claims 1 to 7, **characterized in that** the heat transfer medium heated in the third heat exchanger (W3) is fed to the first heat exchanger (W1) and returned to the third heat exchanger (W3) via a circuit line (L11, L12)

9. Process according to one of the claims 1 to 8, **characterized in that** the second heat exchanger is separately heated.

10. Process according to one of the claims 1 to 9, **characterized in that** the residence time of the fermentation substrate in the first fermenter (F1) depends on the composition of the fermentation substrate, especially the DS content.

11. Process according to one of the claims 1 to 10, **characterized in that** the DS content of the fermentation substrate is set to a value of under 10% by the addition of fermentation liquid and/or water.

12. Process according to one of the claims 1 to 11, **characterized in that** the fermentation substrate separated out is divided into a liquid and a higher concentrated phase before being conducted into the first heat exchanger (W1), the ammonia concentration of the liquid phase only is subsequently reduced and, finally, the liquid phase and the higher concentrated phase are re-combined.

## Revendications

1. Procédé pour la fabrication de biogaz à partir de substrat de fermentation dans un fermenteur ou plus, pour lequel, pendant la durée de séjour du substrat de fermentation dans le fermenteur (F1), le substrat de fermentation est purgé et pompé dans un circuit fermé par un premier échangeur de chaleur (W1) en étant réchauffé jusqu'à 110°C pour être ensuite amené dans un second échangeur de chaleur supplémentaire (W2) en continuant à être réchauffé jusqu'à 180°C, et le substrat de fermentation réchauffé est pompé dans un réservoir fermé ou séparateur (B1) duquel l'ammoniaque est évacué, et le substrat de fermentation à teneur en ammoniaque réduite est évacué du réservoir ou séparateur (B1) et ramené au substrat de fermentation contenu dans le fermenteur (F1), **caractérisé en ce que** la concentration en ammoniaque du substrat de fermentation est surveillée dans le fermenteur (1) et, pendant la fermentation, la teneur en ammoniaque du substrat de fermentation est comprise entre 0,6 et 0,9 g/l et le substrat de fermentation est purgé lorsqu'une valeur limite de 1 g/l est atteinte, le substrat de fermentation purgé est réchauffé avant son introduction dans le réservoir fermé ou séparateur (B1) et comprimé à une pression comprise entre 1,5 et 10 bar et le substrat de fermentation chaud et comprimé est introduit dans le réservoir ou séparateur (B1), au cours du maintien en pression ou du relâchement, il se dégage du substrat de fermentation en plus de l'ammoniaque du CO₂, des traces de H₂S et de la vapeur d'eau qui sont purifiés et évacués sous forme de mélange gazeux par une conduite de gaz d'échappement (L8) intégrée dans le réservoir ou séparateur (B1), l'ammoniaque et le H₂S étant liés ensemble chimiquement sous forme d'engrais dans un épurateur.

2. Procédé selon la revendication 1, **caractérisé en ce que** la quantité de substrat de fermentation introduite dans le réservoir (B1) et la quantité de substrat de fermentation à teneur en ammoniaque réduite évacuée du réservoir (B1) sont adaptées l'une à l'autre de sorte que le réservoir (B1) est rempli en continu d'un volume de substrat de fermentation d'au moins 20 %.

3. Procédé selon l'une quelconque des revendications 1 ou 2, **caractérisé en ce que** la fermentation est réalisée dans deux ou plusieurs fermenteurs montés en parallèle ou en série, le substrat de fermentation étant purgé du premier fermenteur et/ou au début et/ou pendant la fermentation dans un fermenteur placé en amont, et le substrat de fermentation à teneur en ammoniaque réduite est ramené dans un fermenteur ou plus pour poursuivre la fermentation anaérobie.

4. Procédé selon l'une quelconque des revendications 1 à 3, **caractérisé en ce que** la surveillance de la concentration en ammoniaque dans le fermenteur est réalisée en mesurant la teneur en ammoniaque du biogaz évacué du fermenteur.

5. Procédé selon l'une quelconque des revendications 1 à 4, **caractérisé en ce que** le substrat de fermentation purgé est comprimé à une pression comprise entre 2 et 6 bar.

6. Procédé selon l'une quelconque des revendications 1 à 5, **caractérisé en ce que** la concentration en ammoniaque du fermenteur est réglée par la quantité de substrat de fermentation évacuée du circuit et la quantité réintroduite de substrat de fermentation à teneur en ammoniaque réduite.

7. Procédé selon l'une quelconque des revendications 1 à 6, **caractérisé en ce que** le substrat de fermentation à teneur en ammoniaque réduite, évacué du réservoir (B1), est guidé à travers un troisième échangeur de chaleur (W3) afin d'être refroidi à la température du fermenteur.

8. Procédé selon l'une quelconque des revendications 1 à 7, **caractérisé en ce que** l'agent caloporteur réchauffé dans le troisième échangeur de chaleur (W3) est amené au premier échangeur de chaleur (W1) par l'intermédiaire d'une conduite de circuit (L11, L12) et revient ensuite dans le troisième échangeur de chaleur (W3).

9. Procédé selon l'une quelconque des revendications 1 à 8, **caractérisé en ce que** le second échangeur de chaleur est chauffé séparément.

10. Procédé selon l'une quelconque des revendications 1 à 9, **caractérisé en ce que** la durée de séjour du substrat de fermentation dans le premier fermenteur (F1) dépend de la composition du substrat de fermentation, notamment de sa teneur en matière sèche.

11. Procédé selon l'une quelconque des revendications 1 à 10, **caractérisé en ce que** la teneur en matière sèche du substrat de fermentation est réglée en ajoutant du jus de fermentation et/ou de l'eau jusqu'à obtention d'une valeur inférieure à 10 %.

12. Procédé selon l'une quelconque des revendications 1 à 11, **caractérisé en ce que** le substrat de fermentation purgé est séparé en une phase liquide et une phase hautement concentrée avant son introduction dans le premier échangeur de chaleur (W1), à la suite de quoi seule la concentration en ammoniaque de la phase liquide est réduite, et pour finir la phase liquide et la phase hautement concentrée sont réunies.
